Europäisches Patentamt

(19) European Patent Office

Office européen des brevets

(11) Publication number: **0 048 123**
**A1**

## (12) EUROPEAN PATENT APPLICATION

(21) Application number: 81304093.8

(22) Date of filing: 08.09.81

(51) Int. Cl.³: **C 08 L 5/04**
**A 23 L 1/04**

(30) Priority: 12.09.80 US 186779

(43) Date of publication of application:
24.03.82 Bulletin 82/12

(84) Designated Contracting States:
DE FR GB

(71) Applicant: MERCK & CO. INC.
126, East Lincoln Avenue P.O. Box 2000
Rahway, New Jersey 07065(US)

(72) Inventor: Joh, Yongkeun
106 Judy Drive
Keasbey New Jersey 08832(US)

(74) Representative: Crampton, Keith John Allen et al,
D YOUNG & CO 10 Staple Inn
London WC1V 7RD(GB)

(54) **Gelled algin emulsions.**

(57) Gels prepared from algin emulsions containing emulsifiers such as acetylated monoglyceride, and oils or fats which are treated with divalent or trivalent metal ions such as calcium ions. These can be used as water insoluble coatings, films with particular water and gas barrier properties or as impression molds.

EP 0 048 123 A1

Croydon Printing Company Ltd.

-1- K-2023

## TITLE OF THE INVENTION

### GELLED ALGIN EMULSIONS

## BACKGROUND OF THE INVENTION

It is known that the alginates react with certain metal cations, most notably with calcium ions, to produce products which are gels, water insoluble fibers, or fibers. (Industrial Gums, R. Whistler, 2nd ed., Academic Press (1973, pp 56-57). The various products produced utilizing this reaction have included, e.g., meat analog systems (U. S. Pat. 3,919,435), artificial adipose tissue (U. S. Pat. 3,658,550), and food coatings U. S. Pat. 3,395,024 and 3,991,218).

## SUMMARY OF THE INVENTION

It has now been found that gels prepared from algin emulsions containing emulsifiers and oils (or fats) can be used as coatings or films which exhibit improved barrier properties or can be utilized in preparing impression molds with enhanced unmolding properties and impression detail. The gels are prepared by treating the algin emulsions with di- or tri-valent metal ions.

DETAILED DESCRIPTION

The algins useful in this invention are the water soluble salts of alginic acid. Algin is found in all species of Phaeophyceae. The preferred forms are sodium, potassium, and ammonium alginates and the ester, propylene glycol alginate. The algins are commercially available (e.g., KELCOLOID®, KELGIN®, KELMAR®, KELCOGEL®, etc.) from Kelco Division of Merck & Co., Inc., San Diego, California.

The metal ions can be provided by various means, however, a readily water-soluble salt of calcium, preferably $CaCl_2$, in aqueous solution is most desired. It will be recognized that other divalent and trivalent metal ions could be used in the practice of this invention but when the end product is to be used in humans or animals, calcium ions are preferred. Sources of calcium ions include, but are not limited to calcium acetate (monohydrate), calcium phosphate (anhydrous, and monohydrate), dicalcium phosphate (dihydrate, and anhydrous), tricalcium phosphate, calcium sulfate (dihydrate, and anhydrous), calcium gluconate, calcium lactate, calcium citrate, and calcium tartrate.

The emulsifiers useful in this invention are those which contain both lipophilic and hydrophilic groups. Preferably, the emulsifiers are liquid at room temperature. Specific examples of such emulsifiers are acetylated monoglycerides, lecithin, monoglycerides, diglycerides, and their derivatives, polyglycerol esters of fatty acids, polyglycerol oleates, and blends of the above emulsifiers.

Preferred are the acetylated monoglycerides, Myvacet 9-45 and Myvacet 9-40 (E. Kočak).

The gelled algin emulsions of this invention also contain fats or oils. These are preferbly liquid or have a smooth, plastic texture at room temperature. They include oils from vegetables (e.g., corn, olive, and soy), seeds (e.g., cotton, sunflower, and linseed), legumes (e.g., peanut), nuts (e.g., almond and palm kernel), fats from animal sources (e.g., lard, butter, tallow, and fish), hydrogenated oils (e.g., shortenings), and mineral oil.

The concentrations of the ingredients can be varied depending on the properties desired in the final product. Generally, the following amounts are used based on finished product:

|            | % (wt/wt.)     |
|------------|----------------|
| Algin      | 0.05 - 5.0     |
| Emulsifier | 0.05 - 25.0    |
| Oil/fat    | 0.05 - 20.0    |
| Metal ions | 0.0001 - 0.50  |

High levels of emulsifier and oil/fat will improve barrier properties, in general, but an excess will decrease the gel strength of the continuous phase, which will decrease the strength of the films or coatings.

-4-                    K-2023

The gelled algin emulsions of this invention may also contain various optional ingredients such as:

1. Texture modifiers/fillers - dextrins (more turbid), sugars (more brittle), syrups (stickier and softer), starches (more turbid and less brittle), milk solids, diatomaceous earth, clay, and other soluble and insoluble solids.

2. Buffers (e.g. sodium citrate).

3. Sequestrants (e.g., TSPP, sodium hexametaphosphate, and sodium phosphate). It is essential, if using water containing polyvalent metal ions to prepare the algin solution, to use a sequestrant to prevent premature cross-linking of the algin solution.

4. Wetting agents for better spreadability for film casting and to form finer emulsions. A preferred agent is dioctyl sodium sulfosuccinate (DSS), which is waxy at room temperature. DSS is available from American Cyanamid (Pearl River, N.Y.). It is also available as Complemix, a U.S.P. 50% aqueous beverage grade ethanol solution.

5. Flavoring and coloring agents.

These optional ingredients are preferably added in the following procedure prior to the addition of the emulsifier.

The gelled algin emulsions are prepared by:

1) Making an aqueous algin solution.

2) Making an oil (fat)/water emulsion. This is done by slowly adding oil (fat) into the algin solution under high shear.

3) Adding thereto the emulsifier under high shear. The emulsifier is added after the emulsion is formed.

4) When a very fine emulsion is desired, homogenation or colloid milling is recommended. The emulsion may require de-aeration.

5) Making an aqueous divalent or trivalent metal ion solution. A 5% (by weight) $CaCl_2$ (anhy.) solution works well, although solutions in the range of 0.5-10% may be used.

6) Combining the emulsion and ion solutions.

The gelling procedure (addition of the calcium solution to the emulsion) will vary depending on the product to be formed. The general procedure for preparing coatings or films is to first apply the emulsion followed by the application of the ion solution (or the ion solution first followed by the emulsion). If it is desired to use only a one-application procedure, a slowly-dissolving calcium salt may be mixed with the emulsion. This mix is then used to prepare the film or coating. The rate of dis-solution may be enchanced by the use of fumeric, adipic, citric, or like acids.

The coating of this invention can be applied by any of various means. The specific method would depend largely on the nature and size of the object being coated. A revolving pan can be used with or without forced (heated) air. The calcium solution is sprayed on the object, followed by spraying the algin emulsions. (This can be done in reverse order.) A second calcium solution may be sprayed over the algin emulsion or the entire sequence can be repeated to build up thickness. As an alternative to spraying the object may be dipped.

Films, which differ from coatings in that they are flat rather than contoured about an object, are prepared by casting the algin emulsion on a planar surface (preferably clean and smooth) and spraying with the calcium solution, followed by drying. Alternatively, an algin emulsion can be cast containing slowly dissolving calcium salts and slowly dissolving acids (fumaric, adipic). Or an algin emulsion is cast which contains slowly dissolving calcium salts. This is sprayed with an acidic solution (e.g., mild citric or other acids).

Casting can be done by a hand casting bar, a mechanically driven film applicator, a film casting knife, or a continuous casting bar.

After drying the film can be rolled, cut, etc., for subsequent use as a flexible packaging wrap as for food, pharmaceuticals, or any other items where a protective water or air barrier is necessary.

Algin emulsions containing slowly-dissolving calcium salts can be poured onto impression molds and allow to set. The impression molds made from gelled algin emulsions exhibit improved unmolding properties, have a more elastic texture, show greater detail and dry slower than regular algin/calcium molds.

The substantially continuous coating of this invention finds applications in many industries. Of particular importance is the food industry, where it is usually desired to either keep moisture in, e.g. to retard dehydration, or to keep moisture out so as to retard spoilage, loss of color, flavor, etc. The following is a listing of suggested uses for such a coating.

1. Prepared meat products (sausages, extruded meat snacks such as beef jerky, meatballs, pet foods, etc.).

    a. Good water, oxygen barrier properties for reducing dehydration and oxidative flavor degradation;

    b. relatively poor fat barrier which is desirable for fat release during cooking;

    c. colors, flavors (water soluable and/or oil soluble) can be trapped in the coating;

    d. coating is easily applied to different shapes and becomes part of the product as a skin;

e.  barrier properties (water, oxygen and oil) can be adjusted easily by changing acetylated monoglyceride, vegetable oil, algin, or water concentrations.

2.  In fish (fresh and frozen), red meat carcass, and poultry (salmon, beef, pork and lamb carcass, pre-cut steaks, chicken, turkey, etc.)

  a.  Water, oxygen barrier properties for reducing drip loss, surface microbial contamination, oxidative flavor degradation, freezer burn and dehydration.

3.  In cheeses and cheese products (individually wrapped cheeses, soft texture cheese, extruded cheese snacks, loaf or block cheeses, etc.)

  a.  Replaces wax coating - less labor intensive, no cracking of wax and no peeling-off;

  b.  retains desirable shapes for soft cheeses as an edible packaging material;

  c.  reduces dehydration and oxidative flavor degradation of cheese products which are usually not waxed.

4.  Fruits and vegetables - fresh, frozen and dehydrated (frozen fruits and vegetables, blueberries, bananas, cucumbers, apples, tomatoes).

  a.  Prevents freezer burn and oxidative flavor degradation and discoloration of frozen fruits and vegetables;

  b.  replaces wax coatings;

c.    extends freshness of fresh fruits and vegetables for which wax cannot be used, in conjunction with controlled atmosphere storage;

d.    flexibility, allowing incorporation of fungicides, antioxidants (e.g. bananas, dehydrated figs, etc.);

e.    retains shape and gives extra rigidity to delicate and soft-textured fruits and vegetables (e.g. blueberries, cherries, grapes, tomatoes, etc.).

5.  Confections (e.g., liquid-center type candies).

a.    Retains viscous solutions inside confections, whether introduced as liquids or as sugar/invertase enzyme mixtures which subsequently become liquid due to enzyme action;

b.    the coating becomes part of the candy as well as a packaging material;

c.    other wrappers (e.g., aluminum laminates), or additional coatings (e.g., Shellac or sugar coatings) can be applied over the algin emulsion/calcium coating.

6.  Pharmaceutical products

a.    Useful as a subcoating prior to pan coating of tablets or pills;

b.    enteric coatings, sustained release pills, etc.;

c.    liquid-containing pharmaceutical candies such as cough syrups, mouth freshner, etc..

-10-                               K-2023

7.  Edible/flexible packaging film for other food
    products and pharmaceuticals.


        Due to the thermal irreversability and
non-sticky nature of this novel coating a multitude
of other applications will be apparent to the
practitioner in the food, pharmaceutical, industrial,
and commercial fields.

        The invention is further defined by
reference to the following examples which are
intended to be illustrative and not limiting.


                    EXAMPLE 1
                Coating For Meat Balls


| Ingredient*                    | Gram  |
|--------------------------------|-------|
| Water                          | 158.7 |
| Veg. Oil                       | 30.0  |
| Myvacet 9-45®                  | 10.0  |
| Dextrin (Fro Dex 10®)          | 10.0  |
| KELGIN LV®                     | 1.0   |
| Calgon®                        | 0.3   |
| Flavor (to suit)               |       |
| Color (to suit)                |       |
|                                |       |
|                        Total   | 210.0 |


*Complemix® (10-15 ppm) may be added for improved
adhesion of the coating.

Procedure

1. Make meat ball; fresh or cooked.

2. Spray or dip the meat ball in 5% $CaCl_2$ solution.

3. Remove excess $CaCl_2$ solution by forced air.

4. Dip or spray the meat ball in the above algin emulusion.

5. Blow off excess algin emulsion.

6. Dip or spray the meat ball again in $CaCl_2$ solution (5%) if a stronger or shinier surface is desired.

Characteristics

1. Translucent

2. Not sticky on surface.

3. Soft, flexible, not chewy.

EXAMPLE 2

Coating For Camembert Cheese

| Ingredient* | Gram |
| --- | --- |
| Water | 157.7 |
| Veg. Oil | 30.0 |
| Myvacet 9-45® | 10.0 |
| KELGIN LV® | 2.0 |
| Calgon | 0.3 |
| Flavor (to suit) | |
| Color (to suit) | |
| Total | 200.0 |

*Complemix® (10-15 ppm) may be added for improved adhesion of coating.

Procedure

1. Chill cheese if too soft for easy handling.
2. Dip or spray cheese pieces (slice or ball) in 5% $CaCl_2$ solution.
3. Remove excess $CaCl_2$ solution by forced air.
4. Dip or sprayin the algin emulsion.
5. Blow off excess algin emulsion.
6. Dip or spray the cheese in $CaCl_2$ solution algin.

Characteristics

1. Translucent (whitish)
2. Soft; similar to the texture of cheese
3. Sheen on surface
4. Non-sticky surface.

EXAMPLE 3

Coating for Crown of Banana

| Ingredient | Gram |
|---|---|
| Water | 181.698 |
| Veg. Oil | 10.000 |
| Myvacet 9-40 | 5.000 |
| KELGIN MV® | 2.000 |
| Calgon | 0.300 |
| Complemix® | 0.002 |
| Fungicide (to suit) | |
| Total | 200.000 |

Procedure

1  Dip or spray the crown of bananas in 5% CaCl$_2$ solution.

2.  Remove excess CaCl$_2$ solution by forced air.

3.  Dip or spray in algin emulsion.

4.  Dip or spray in CaCl$_2$ bath algin if desired.

Characteristics

1.  Transparent

2.  Wet appearance

## EXAMPLE 4

### Coating for Tablets or Candies (I)

| Ingredient | % |
|---|---|
| Part I | |
| Water | 45.21 |
| Sugar (granular) | 37.14 |
| Dextrin (DE 10) | 8.07 |
| Mineral Oil | 4.85 |
| Myvacet 9-45® | 3.23 |
| KELCOGEL LV® | 0.97 |
| Calcium Sulfate dihydrate | 0.52 |
| Tetra sodium pyrophosphate (TSPP) | 0.01 |
| | 100.00 |

Part II

2.5% acetic acid solution

Procedure

1. Add TSPP to water

2. Blend KELCOGEL LV with dextrin and 1/3 of sugar and sift into water under good agitation.

3. After the gum is hydrated, add remaining sugar.

4. Add mineral oil slowly with high mixing.

5. Make slurry of calcium sulfate dihydrate in Myvacet 9-45, and add to the batch under high shear mixing. Homogenize (or colloid mill) is optional for very tight emulsion. Deairate the emulsion if necessary.

6. In revolving pan with warm forced air gum, spray algin emulsion on objects.

7. After the object is evenly coated with algin emulsion, continue to rotate pan with warm forced air until the surface of object become tacky/partially dry.

8. Spray with acetic acid solution and continue to rotate pan with warm forced air. (Acetic acid can be sprayed first and algin emulsion later.)

9. Repeat the spraying procedures (6 to 8) for thicker coating.

## EXAMPLE 5

### Coating for Tablets of Candies II

| Ingredients | % |
|---|---|
| Part I | |
| Water | 45.39 |
| Sugar (granules) | 37.00 |
| Dextrin (DE 10) | 8.10 |
| Mineral Oil | 5.00 |
| Myvocet 9-40® | 3.50 |
| KELCOGEL LV® | 1.00 |
| Tetrasodium pyrophosphate (TSPP) | 0.01 |
| | |
| Total | 100.00 |

Part II

50% calcium chloride anhydrous ($CaCl_2$) solution

Procedure

1. Add TSPP in water.
2. Blend KELCOGEL LV with dextrin and 1/3 of sugar, and sift into water under good agitation.
3. After the gum is fully hydrated, add remaining sugar.
4. Add mineral oil slowly with high shear mixing
5. Add Myvacet 9-40 slowly with high shear mixing. Homogenize (or colloid mill) is optional for very tight emulsion. Deairate the emulsion if necessary.

6. In revolving pan with forced air gum (warm), spray $CaCl_2$ solution on objects.

7. After CaCl$_2$ solution is partially dried, spray algin emulsion.

8. Rotate pan under forced air until the surface becomes tacky to partially dry.

9. Spray CaCl$_2$ and rotate pan until the surface becomes dry.

10. Repeat steps 6 to 8 for building up the thickness coating.

Characteristics of Coatings

1. White, brittle and hard.

2. Smooth surface.

3. Slippery surface when wet with water.

4. In water will swell slightly but not disintegrate.

EXAMPLE 6

Impression Mold

| Ingredient | Wt(g) |
|---|---|
| Part I | |
| Water | 100.000 - 150.000 |
| Diatomaceous earth* | 30.000 |
| Mineral Oil | 5.000 |
| Improved KELMAR® | 2.500 |
| Complemix® | 0.002 |
| Part II | |
| Calcium sulphate dihydrate | 2.500 |
| Myvacet 9-45® | 5.000 |

*Other fillers such as MgCO$_3$ or other pure, inert material may be used.

Procedure

1. Thoroughly blend diatomaceous earth and improved KELMAR, and add to water under good mixing.

2. Add mineral oil and Complemix® with good mixing.

3. Make slurry of calciuim sulphate dihydrate in Myvocet 9-45®.

4. Mix rapidly calcium sulphate dihydrate slurry (3.) with algin emulsion (2.).

5. Take the impression.

6. After approximately 4 min. the mold is ready to be removed.


Characteristics

1. Easy to unmold.

2. Sheen; smooth surface.

3. Fine detail impression.

4. Impression mold dries slower than regular algin impression.

## WHAT IS CLAIMED IS:

1. A gelled algin emulsion comprising 0.05 to 5.0 weight % algin, 0.05 to 25 weight % emulsifier, 0.05 to 20 weight % oil or fat, and 0.0001 to 0.5 weight % divalent or trivalent metal ions.

2. A gelled emulsion of Claim 1 wherein the algin is sodium alginate, potassium alginate, ammonium alginate, or alkylene glycol alginate and the ions are calcium.

3. A gelled emulsion of Claim 2 wherein the emulsifier is acetylated monoglyceride, lecithin, monoglyceride, diglyceride, mono- or diglyceride derivatives, polyglycerol esters of fatty acids, or polyglycerol oleates, the oil is corn, soy, olive, fish, cottonseed, linseed, sunflower, peanut, nut, mineral oil, or hydrogenated oil, and the fat is butter, lard, or tallow.

4. A gelled emulsion of Claim 3 wherein the emulsifier is acetylated monoglyceride.

5. A process for forming a substantially continuous moisture resistant coating on objects which comprises sequentially coating said object with
   a. an aqueous emulsion comprising algin, emulsifier, and oil or fat, and

b.   a 0.5-10 weight % aqueous di- or tri-valent metal ion solution.

6.   A process for forming a moisture resistant film which comprises

   a.   casting on a planar surface an aqueous emulsion comprising algin, emulsifier, and oil or fat, and then

   b.   coating said emulsion with a solution of di- or tri-valent metal ions.

7.   A process for forming an impression mold which comprises a) preparing a mold composition comprising algin, emulsifier, oil or fat, a slowly soluble calcium salt, and filler and b) making an impression in said composition.

**European Patent . Office**

# EUROPEAN SEARCH REPORT

Application number

EP 81 30 4093

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | CHEMICAL ABSTRACTS, vol.80, no. 2, January 21, 1974 page 337, abstract 13833a Columbus, Ohio, US & JP - A - 73 14064 (NIWANE HICHIRO) (02-05-1973) * Abstract * | 1-4 | C 08 L 5/04 A 23 L 1/04 |
| X | GB - A - 586 157 (WILLIAM J.S. PESCHARDT) * Page 2, line 115 - page 3, line 15; page 3, lines 55-57; claims * | 1,2,5, 6 | TECHNICAL FIELDS SEARCHED (Int. Cl.³) |
| | US - A - 3 989 220 (ALLEN A. GREENBERG) * Column 3, lines 12-27 * | 1,7 | C 08 L 5/04 A 23 B 4/10 A 23 D 5/00 A 23 L 1/04 |
| | US - A - 3 255 021 (ROLAND D. EARLE) * Claims * | 1,5 | |
| | FR - A - 874 977 (ALBERT-ANTOINE POULVEREL) * Page 2, lines 44-50; abstract * | 1 | CATEGORY OF CITED DOCUMENTS |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search The Hague | Date of completion of the search 15-12-1981 | Examiner LENSEN |
|---|---|---|

EPO Form 1503.1   06.78